# EUROPEAN PATENT APPLICATION

(11) **EP 2 298 938 A1**
(43) Date of publication of application: **23.03.2011**
(21) Application number: 10181397.0
(22) Date of filing: 21.09.2005
(51) Int. Cl.: C12Q 1/70, C12N 7/00, C12N 7/02, C12N 7/04, C12N 7/08

(54) **Porcine reproductive and respiratory syndrome isolates and methods of use**

(30) Priority: 21.09.2004 US 611824 P; 23.12.2004 US 22262
(62) Divisional of application: 05808396.5
(71) Applicant: Boehringer Ingelheim Vetmedica, Inc., St. Joseph, MO 64506 (US)
(72) Inventor: Roof, Michael, Ames, IA 50014 (US); Vaughn, Eric, Ames, IA 50010-1110 (US); Johnson, Wesley, Ames, IA 50010 (US)
(74) Representative: Hammann, Heinz

(57) **Abstract**

The present invention relates new PRRS virus isolates and attenuated forms thereof which are useful in immunogenc compositions, in particular modified live vaccines..

## Description

### BACKGROUND OF THE INVENTION

### Field of the Invention

The present invention is concerned with new isolated wild-type PRRS virus isolates and corresponding improved attenuated PRRS viruses, the uses of such attenuated viruses in vaccines and immunological compositions, as well as methods of measuring the magnitude of viremia, rate of growth, antibody response, and combinations thereofin such isolates and viruses. More particularly, the present invention provides methods for predicting the virulence of new or previously uncharacterized PRRS isolates, and the attenuation and use of such isolates in vaccines and immunological compositions.

### Description of the Prior Art

Porcine reproductive and respiratory syndrome virus (PRRSV) is an enveloped single stranded RNA virus classified in the family Arteriviridae (Cavanaugh, 1997). It causes a widespread disease of swine that was first described as 'mystery swine disease' in the USA in 1987 (Hill, 1990). The disease manifests as respiratory illness in all age groups of swine leading to death in some younger pigs and severe reproductive problems in breeding age females.

The dynamic nature of PRRSV allows for constant change in the disease and provides ample opportunity for the appearance of new isolates (Andreyev et al., 1997; Murtaugh et al. 1998; Meng, 2000). The fact that PRRSV changes so readily, coupled with its ability to cause devastating problems for swine producers, makes it an important subject for research (Mengeling et al., 1998; Pejsak et al., 1997) and for the development of vaccines and other methods of reducing the effects of infection. Variation in levels of isolate virulence were demonstrated in lung lesions, and death in swine (Halbur et al., 1996), but efforts to link biological and immunological differences to specific genetic differences has been largely unsuccessful (Albina et al., 1998; Key et al., 2001; Yuan et al., 2001; Murtaugh et al., 2002; Grebennikova et al., 2004). Studies examining the safety and efficacy of PRRS vaccines include the work of Labarque et al., (2003), Mengeling et al., (2003a), and Nodelijik et al. (2001). These studies show that under experimental conditions, modified live PRRS vaccines reduce the amount and duration of viremia as well as fever and lung lesions after virulent challenge.

Opriessing et al. (2002) showed that isolates with high amino acid sequence homology in open reading frame 5 (ORF5) caused significantly different levels of pneumonia in pigs. Variation in swine responses to PRRSV also are affected by host variation (Mengeling et al., 2003b). Virulence has been examined in relation to replication rates and distribution of PRRSV in pigs (Haynes et al., 1997), to macrophage copper clearing capabilities (Thanawongnuwech et al.,1998), and the anemia levels of the host animal (Halbur et al, 2002). However, these methods have been deficient in providing effective methods for predicting the virulence of new or previously uncharacterized PRRS isolates.

Accordingly, what is needed in the art is a method of predicting the virulence of PRRS isolates. What is further needed in the art is a method of predicting the virulence of PRRS isolates based on the rate *of in vivo* PRRS viral growth and/or viremia magnitude in a swine after administration or exposure of the isolate to a previously PRRS-free swine.

### SUMMARY OF THE INVENTION

The present invention overcomes the problems outlined above and provides new isolated wild-type PRRS virus isolates, attenuated versions of such viruses, and corresponding vaccines comprising the attenuated PRRS viruses in pharmaceutical compositions. In more detail, the wild-type viruses of the invention are denominated SDSU 73, 17198-6 and MN 184, and mixtures thereof. Attenuated versions of these virus isolates, and complete vaccines comprising the attenuated isolates are capable of conferring *in vivo* immunity against PRRS in swine, without generation of significant PRRSV clinical signs.

Another feature of the attenuated PRRS isolates of the invention is that they retain a substantial proportion of the viremia found in the wild, unattenuated viruses. Thus, the attenuated viruses exhibit at least about 50%, and more preferably at least about 75%, of the viremia exhibited by the wild unattenuated viruses. In this way, the attenuated viruses are capable of conferring a more rapid and complete PRRS immunity in swine.

The wild viruses can be attenuated by a variety of methods, such as repeated serial passage in cell culture, gene insertion, gene switching, gene deletion, base-pain substitution, and temperature-sensitive mutation. Such attenuated viruses may then be used in a variety of immunological compositions including vaccines using methods that are well known in the art. In some forms, these attenuated viruses may further be killed or inactivated using methods that are well known in the art, prior to incorporation or inclusion in an immunogenic composition according to the present invention. Examples of immunogenic compositions useful for preventing PRRS infection or reducing the severity of clinical signs of PRRS infection include vaccines made with Abst-1 or JA-142.

It has also been discovered that the vaccines of the invention may be stored in a frozen condition until ready for use, without detracting from the desirable characteristics of the vaccines. This addresses a continuing problem in the art, inasmuch as prior PRRS vaccines have not been suitable for frozen storage.

The present invention further provides methods for predicting the degree of virulence of a new or uncharacterized PRRS virus isolate. Such methods generally involve assessing a new or previously uncharacterized isolate of PRRS for at least one of the following parameters: rate of viral growth; magnitude of viremia; antibody response; or combinations thereof. The results of such an assessment are then used to predict the degree of virulence of the new or previously uncharacterized isolate. The methods generally involve administering or exposing a PRRS-free swine to a quantity of the new or uncharacterized PRRS isolate and allowing the virus to replicate for a period of up to about 15 days, more preferably from about 2-12 days, still more preferably from about 3-10 days and still more preferably from about 3-7 days. The mode of administration can be accomplished using any conventional manner, including oral, intranasal, intramuscular, intra-lymph node, intradermal, intraperitoneal, subcutaneous, and combinations thereof, but most preferably through intranasal administration. The amount of the dose for intranasal administration is preferably up to about 5 ml, still more preferably between about 0.5 ml to about 4 ml, still more preferably between about 1 ml and about 3 ml, and still more preferably about 2 ml. The concentration of virus in each dose should be up to about 5.0 Log₁₀ TCID₅₀/ml,more preferably between about 1.0 to about 4.0 Log₁₀ TCID₅₀/ml, and still more preferably about 2.5 to 3.5 Log₁₀ TCID₅₀/ml, and most preferably about 3.0 Log₁₀ TCID₅₀/ml. At a selected time or times during this replication period, biological samples are taken from the swine and measurements of the rate of growth of the administered virus, viremia magnitude, antibody response, and/or combinations thereof are taken. Data gathered from these measurements is then compared with the rate of growth, viremia magnitude, antibody and/or combinations thereof for a known and charaterized isolate, as a measure of predicted virulence of the new or unknown isolate.

Using the methods of the present invention, the rate of PRRS viral growth, viremia magnitude, antibody response, and/or combinations of these characteristics were measured in swine which had one of eight different PRRSV isolates administered thereto. Each of these isolates had a known level of virulence and clinical disease manifestations. These same characteristics were also measured in swine which had a combination of all eight isolates administered thereto.

More specifically, one hundred (100) healthy two-three week old pigs were divided randomly by weight into ten groups with each group having ten pigs. All pigs were tested for PRRS infection using HerdChek ® PRRS ELISA 2XR (IDEXX Laboratories Inc., Westbrook, ME). Eight of the groups received an administration of one of the eight isolates, one group received an administration consisting of a combination of all eight isolates, and the last group received an administration Eagle's Minimum Essential Medium (EMEM) to act as a control group. A sample of each viral inoculation was retitrated for titer confirmation. Preferably the titer of the administered virus is designed to mimic a natural exposure level of virus. Biological samples in the form of blood were collected at various times throughout the experiment. Each sample was analyzed by virus isolation, quantitative reverse transcriptase-polymerase chain reaction (RT-PCR), HerdChek ® PRRS ELISA 2XR, and PRRSV protein-specific ELISA.

Virus isolation was performed on CL2621 (an MA 104 cell line) cells by serially diluting serum and combining it with EMEM, gentamicin (Sigma Chemical Co., St. Louis, MO) and Fungizone (Invitrogen Corp., Grand Island, NY). The dilutions were then incubated and examined for cytopathic effect (CPE). The Reed-Muench calculation was used to determine titers.

RT-PCR was performed using the QIAamp Viral RNA Mini-Kit ® (Qiagen, Inc., Valencia, CA) and PRRSV was detected using a single-tube assay by Tetracore, Inc. (Gaithersburg, MD). To determine virus quantitation, a standard curve was developed and concentrations of the unknown samples were determined by linear extrapolation of the cycle threshold values plotted against the known concentration of the 3' UTR transcript product.

Antibody measurement using ELISA S/P ratios were generated using HerdChek ® PRRS ELISA 2XR using the manufacturer's instructions. PRRSV protein-specific ELISA was performed using recombinant isolate VR2332 nucleocapsid (N) and non-structural protein 4 (nsp 4) expressed in BL21 (DE3)-RP cells (Stratagene, La Jolla, CA).

All pigs were weighed at the beginning and at the end of the study. Additionally, on every day of the study, each pig was evaluated and scored by a veterinarian for clinical signs of PRRS disease. All resulting data was analyzed statistically and compared on a group-by-group basis.

Nine of the ten groups were subsequently challenged a second time with a virus isolate of high virulence to allow further evaluation of levels of homologous protection and vaccine versus wild type heterologous protection (live virus exposure concept). Following this challenge, the animals were evaluated and necropsied for analysis using virus isolation and immunohistochemistry.

The present invention therefore also provides an immunogenic composition comprising an attenuated PRRS virus isolate. In preferred forms, the composition can also include a pharmacologically compatible carrier and/or an adjuvant. One example of such a composition would include Abst-1 and/or an attenuated form of a PRRS virus that is predicted to be virulent by the methods described above.

The present invention also provides an improved method of selecting a PRRS virus isolate for attenuation and inclusion in an immunogenic composition. The method generally includes the steps of a) obtaining a PRRS isolate of unknown virulence; b) administering a quantity of said PRRS virus isolate into a PRRS-free swine; c) allowing said isolate to replicate in the swine for a period of from about 3-15 days; d) measuring the rate of virus growth and/or the magnitude of viremia during said period; e) comparing said rate of growth or viremia magnitude with the rate of growth and/or viremia magnitude of a PRRS virus isolate of known virulence; and f) selecting an isolate for attenuation and inclusion into an immunogenic composition based on its rate of growth and/or its viremia magnitude in comparison to isolates of known virulence. It would be preferred to select an isolate that was predicted to be highly virulent for inclusion in an immunogenic composition although isolates predicted to be of lessor virulence including those rpedicted to be avirulent could be used. This is because the isolates predicted to be of higher virulence had higher growth rates and/or magnitudes of viremia, which generally induce a more vigorous and protective immune repsonse. By using the methods of the present invention, the selection of which isolates to use for inclusion in immunogenic compositions will be simiplified and the likelihood of having a useful vaccine that protects against the virulent PRRS isolates will be increased.

As used herein, "rate of growth" refers to the measurement of virus replication over time in swine. Preferred examples of this measurement are provided in Example 1. "Viremia magnitudes" as used herein, refers to the concentration of virus circulating in the blood of swine. Preferred examples of this measurement are also provided in Example 1.

### BRIEF DESCRIPTION OF THE DRAWINGS

Figure 1 is a graph of mean serum virus titer versus time expressed as log₁₀ TCID₅₀/ml for the swine test of Example 1;
Fig. 2 is a graph of mean PRRSV virus concentration in serum in the swine test of Example 1, as measured by real time RT-PCR;
Fig. 3 is a graph of mean S/P ratios versus time using a commercial ELISA assay;
Fig. 4 is a graph illustrating repeated measures analysis for the commercial ELISA assay and the log₁₀ TCID₅₀/ml data of Example 1, wherein the group average under the ELISA S/P ratio curve was plotted against the group average area under the log₁₀ TClD₅₀/ml;
Fig. 5 is a graph illustrating repeated measures analysis for the commercial ELISA assay an RT-PCR concentration data of Example 1, wherein the group average area under the ELISA ratio curve was plotted against the group average area under the RT-PCR concentration curve;
Fig. 6a is a graph of absorbance versus time for Example 1;
Fig. 6b is a graph of absorbance vs. time, illustrating the effect of PRRSV isolate on nsp-4 IgG response, wherein the data are the mean values of 10 animals, except where animals died;
Fig. 7 is a graph illustrating repeated measures analysis using the nsp-4 and log₁₀ TCID₅₀/lml data of Example 1, wherein the group average area under the nsp-4 curve was plotted against the group average area under the log₁₀ TCID₅₀/ml curve;
Fig. 8 is a graph of repeated measures analysis using the clinical scores and log₁₀ TCID₅₀/ml data of Example 1, wherein the group average under the clinical scores curve was plotted against the group average area under the log₁₀ TCID₅₀/ml curve; and
Fig. 9 is a graph of the IHC scores for each isolate in this application.

### DETAILED DESCRIPTION OF THE PREFERRED EMBODIMENTS

The following examples set forth preferred isolates and procedures in accordance with the present invention. It is to be understood, however, that these examples are provided by way of illustration only, and nothing therein should be deemed a limitation upon the overall scope of the invention.

### EXAMPLE 1

### Materials and Methods:

One hundred healthy 2-3 week-old pigs were obtained from a PRRS-free commercial herd and were maintained at Veterinary Resources, Inc., Ames, Iowa, under the supervision of a veterinarian. Animals received food and water *ad libitum.* All of the animal care and laboratory personnel involved with the study were blinded to the treatments given to the various groups of animals. Pigs were tested by HerdChek® PRRS ELISA 2XR (IDEXX Laboratories Inc. Westbrook, ME) to determine if any pigs were infected with PRRSV. All of the pigs for this example tested negative. The pigs were then randomly divided by weight into 10 groups, with 10 pigs per group.

A total of eight PRRSV isolates were used in this example. These isolates have been designated VR-2332, Ingelvac® PRRS MLV, JA 142, Ingelvace® PRRS ATP , SDSU 73, Abst-1, MN 184, and 17198-6. These eight isolates span the history of PRRS disease, exhibit a wide range of virulence levels, and represent relevant clinical disease manifestations. All of the virus isolates grew readily on ATCC cell line CL2621 cells (CL2621 is a proprietary cell line obtained from NVSL, Ames, IA,) (an MA-104 monkey kidney cell line). Three of the primary field isolates, VR-2332, JA-142, and SDSU 73, also had attenuated forms, Ingelvac® PRRS MLV (Boehringer Ingelheim Vetmedica Inc., St. Joseph, MO), Ingelvac® PRRS ATP (Boehringer Ingelheim Vetmedica Inc., St. Joseph, MO), and Abst-1 , respectively. These attenuated forms all exhibit low or undetectable virulence that was derived by *in vitro* passaging to attenuation. The PRRSV isolate ATCC VR-2332 was isolated in 1991 in Minnesota and was used at cell culture passage three. The attenuated form of this virus is commercially available under the trade-name Ingelvac® PRRS MLV. The PRRSV isolate JA 142 (ATCC No. PTA-6504), provided by William Mengeling, National Animal Disease Center, Ames, Iowa, was isolated in 1997 in Iowa from a severe "abortion-storm" case of reproductive failure and was used at cell culture passage five. The attenuated form of JA 142 is commercially sold under the trade-name Ingelvac® PRRS ATP and has been assigned ATCC No. VR-2638. PRRSV SDSU 73 (ATCC No. PTA-6322) was recovered in Iowa from a severe case of reproductive disease in 1996 and was used at cell culture passage one. The attenuated form of SDSU 73, designated Abst-1 (ATCC No. PTA-6320), was obtained by 52 passages. The PRRSV isolate 17198-6 (ATCC No. PTA-6321) was obtained from Oklahoma in 1997 from a herd experiencing severe reproductive disease and was used at passage level four. The PRRSV MN 184 isolate (ATCC No. PTA-6319) was obtained in 2001 from a swine farm experiencing severe reproductive disease and sow mortality in southern Minnesota and was provided by Kurt Rossow, University of Minnesota, St. Paul. This isolate was used at a cell culture passage of one. Additionally, a pool consisting of a combination of all isolates was produced.

On day 0, each of the eight PRRSV isolates and the PRRSV pool were diluted to approximately 3.0 Log₁₀ TCID₅₀/ml in Eagle's Minimum Essential Medium (EMEM) (JRH Bioscience, Lenexa, KS) containing 4% FBS (JRH Bioscience, Lenexa, KS) and administered intranasally to pigs at a dose of 2 ml (1 ml per nostril). The untreated control group received 2 ml of media. The inocula were titrated on 96-well plates containing three-day-old CL2621 cells for titer confirmation using the Reed-Muench method (Reed et al., 1938). The observed titers administered to pigs, together with a description of the virulence level and isolation information, are shown in Table 1.

**Table 1. Virulence and Inoculation Titer of Isolates.**

| Group | Isolate | Year Isolated | Virulence*** | Titer log₁₀ TCID₅₀/ml |
|---|---|---|---|---|
| 1 | VR 2332 | 1991 | Moderate | 3.43 |
| 2 | Ingelvac® PRRS MLV* | USDA license 1996 | Attenuated VR2332 | 3.02 |
| 3 | JA 142 | 1997 | High | 3.13 |
| 4 | Ingelvac®PRRS ATP* | USDA license 1999 | Attenuated JA 142 | 4.14 |
| 5 | SDSU 73 | 1996 | High | 2.75 |
| 6 | Abst-1 * | Attenuated 1999 | Attenuated SDSU 73 | 4.18 |
| 7 | MN 184 | 2001 | High | 4.10 |
| 8 | 17198-6 | 1997 | High | 2.81 |
| 9 | Pool** | N/A | High | 3.71 |
| 10 | Control | N/A | N/A | N/A |

| | | | | |
|---|---|---|---|---|
| * attenuated PRRSV isolates. ** Mixture containing all of the eight isolates *** Summary of lung lesions reported in Symposium on Emerging Diseases, Rome 2003. | | | | |

The isolates were then compared to determine their genetic similarity through an analysis of their percent sequence identity. Sequence identity was determined by submitting virus samples to the University of Minnesota Diagnostic Laboratory for sequence analysis. The results of ORF 5-6 were provided and then compared to a PRRS virus consensus sequence. Individual base pair differences were noted and then the % sequence identity was compared between isolates. As those of skill in the art are aware, blast searching can also be done at various websites. For example, the University of Minnesota provides a PRRSV database (ccgb.umn.edu/cgi-biucommon/web_blast.cgi) that lists sequences from isolates from 1989-2003. Another frequently used site is the NCBI BLAST link found at ncbi.nlm.nih.gov/BLAST.

As shown by the percent sequence identity and the dendogram in Table 2, the virulent field isolates are quite genetically distinct and represented a diverse group of PRRSV isolates. In contrast, the parental and vaccine PRRSV pairs were nearly genetically identical. The pairwise comparison and dendrogram of Table 2 were generated using the Lasergene software suite of sequence analysis tools (DNASTAR, Inc, (Madison, WI)).

**Table 2. Pairwise comparisons of ORF5 nucleotide sequence of virulent and attenuated PRRSV isolates used in the study. Percent similarity is shown in the upper right and percent divergence is shown in the lower left of the table. The dendrogram shows the genetic relatedness of the isolates. The bar indicates 1 nucleotide change per 100 residues. VR2332 is the parent isolate of Ingelvac PRRS MLV, JA 142 is the parent isolate of Ingelvac PRRS ATP and SDSU 73 is the parent isolate of Abst-1.**

| **Percent Identity** | | | | | | | | |
|---|---|---|---|---|---|---|---|---|
| | VR 2332 | Ingelvac PRRS MLV | JA-142 | Ingelvac PRRS ATP | SDSU 73 | Abst-1 | MN 184 | 17198-6 |
| VR 2332 | | 99.7 | 91.0 | 90.5 | 90.0 | 89.6 | 86.4 | 90.4 |
| Ingelvac PRRS MLV | 0.3 | | 90.7 | 90.2 | 9.7 | 89.2 | 6.4 | 90.0 |
| JA-142 | 9.7 | 10.1 | | 99.2 | 92.7 | 92.2 | 87.2 | 92.2 |
| lngelvac | 10.3 | 10.7 | 0.8 | | 91.9 | 91.4 | 86.4 | 91.4 |
| PRRS ATP | | | | | | | | |
| SDSU 73 | 10.9 | 11.3 | 7.8 | 8.8 | | 99.5 | 87.2 | 91.7 |
| Abst-1 | 11.5 | 11.9 | 8.4 | 9.4 | 0.5 | | 86.7 | 91.2 |
| MN 184 | 15.5 | 15.5 | 14.4 | 15.5 | 14.4 | 15.1 | | 86.1 |
| 17198-6 | 10.5 | 10.9 | 8.8 | 9.7 | 9.0 | 9.6 | 15.9 | |

| | | | | | | | | |
|---|---|---|---|---|---|---|---|---|
| **Percent Divergence** | | | | | | | | |

At day 49, 2ml of virulent isolate of PRRS MN 184 was administered intranasally to groups 1-9. The MN 184 isolate was diluted in MEM + 4% FCS, such that the concentration was equal to log 3.0 +/- 0.5 per ml. The diluted challenge virus was titrtaed on 96-well plates containing three day old CL2621 cells for testing. The animals were evaluated for 14 days and then necropsied.

### Evaluation of Viremia

Blood samples were collected from each pig in each group by vacutainer on days 0,1, 3, 7, 15, 21, 28, 35, 42, and 49. Serum was separated from clotted whole blood by centrifugation at 6000 RPM for 20 minutes. Serum samples were then divided for analysis by virus isolation, TCID₅₀ Assay, quantitative reverse transcriptase-polymerase chain reaction (RT-PCR), HerdChek® PRRS ELISA 2XR, and PRRSV protein-specific ELISA. The serum samples in this study were processed immediately after collection and were chilled on ice within 3 hours of being obtained. The samples were stored for a maximum of 24 hr at 4°C and at -70°C thereafter. Serum tested by RT-PCR was frozen at -70°C the day of collection and stored until the testing could be performed at which time only the number of samples that could be tested within 24 hours were thawed, extracted, and tested.

For the TCID₅₀ assay, 100 µl of serum from each pig was added to a dilution tube containing 900 µl of EMEMm + 2% FBS + 50 µg/ml gentamicin (Sigma Chemical Co. St. Louis, MO) + 2.5 µg/ml Fungizone (Invitrogen Corporation, Grand Island NY). This tube was vortexed and 100 µl was transferred to another dilution tube containing 900 µl of EMEMm+ 2% FBS + 50 µg/ml gentamicin + 2.5 µg/ml Fungizone. This process was repeated until a final dilution of 10⁻⁶ was reached. Four replicates for each dilution were plated on 96-well plates, containing the CL2621 (MA 104 cells), 100 µl per well, at 37°C and 4% CO₂ for eight days. Each well then was examined for cytopathic effect (CPE) and the titers were determined using the Reed-Muench calculation. For virus isolations, 100 µl of serum was added to each of the duplicate test wells containing MA 104 cells. The plates were then incubated for one hour at 37°C and 4% CO₂.Next, 500 µl of EMEM + 2% FBS + 50 µg/ml gentamycin + 2.5 µg/ml Fungizone was added to each well. The plates were allowed to incubate at 37°C with 4% CO₂ for eight days and then each well was examined for CPE.

To extract viral RNA from the serum for quantitative RT-PCR, the QIAamp Viral RNA Mini-Kit®(Qiagen Inc. Valencia, CA) was used as described in the kit instructions. For real-time PCR, a commercially available real-time, single-tube, RT-PCR assay for the detection of U.S. PRRSV was provided by Tetracore Inc. (Gaithersburg, MD) and used to detect PRRSV RNA. A minor groove binding (MGB) 5' nuclease probe and primers were designed from the 3' untranslated region (UTR) PRRSV genomic region by alignment of GenBank isolates and based on conserved areas of the 3'UTR primer and probe region. The PRRSV RNA was transcribed in a single tube using a 25 µl reaction volume consisting of Tetracore U.S. PRRSV Master Mix (18.9 µl Master mix, 2 µl Enzyme mix 1, 0.1 µl Enzyme mix 2) and 4 µl of extracted RNA. The reaction tubes were loaded into the Smart Cycler II^{®} block (Cepheid, Sunnyvale, CA) and software settings of fluorescent detection were set for automatic calculation of the baseline with the background subtraction on. The thermal cycler program consisted of 52°C for 1800 seconds, 95°C for 900 seconds, and 45 cycles at 94°C for 30 seconds, 61°C for 60 seconds and 72°C for 60 seconds. A PCR reaction was considered positive if the cycle threshold (Ct) level was obtained at ≤ 45 cycles. For quantitation, known amounts of serially diluted *in vitro* transcript RNA product (1 x 10 ⁻¹ through 1 x 10⁸ copies/µl) were used to generate a standard curve. Copy/ml concentrations of the unknown samples were determined by linear extrapolation of the Ct values plotted against the known concentration of the 3'UTR transcript product.

### Antibody Measurement

ELISA S/P ratios were generated by performing the HerdChek® PRRS ELISA 2XR (INDEXX Laboratories, Westbrook, MA) according to the manufacturer's instructions. PRRSV protein-specific ELISA for The HerdChek® was performed with recombinant isolate VR2332 nucleocapsid (N) and nonstructural protein 4 (nsp 4) which were expressed in BL21 (DE3)-RP cells (Stratagene) from the plasmid pET 24b as fusion proteins containing an amino terminal myc-tag and a carboxyl terminal 6x histidine tag. Denatured proteins were dialyzed in 0.1 M Tris HCI, pH 8.0, 6 M guanidine-HCI, 2 mM EDTA and adjusted to a concentration of 3 mg/ml. DTT was added to 300 mM and the solution was filtered through a 0.45 µm membrane. Reduced protein was added into refolding buffer (100 mM Tris HCI, pH 8.0, 0.5 M L-arginine, 8 mM oxidized glutathione, 2 mM EDTA, 10 µM pepstatin A, 10 µM leupeptin, and 1 mM PMSF), filtered (0.22 µm) and stirred overnight. The purified protein was concentrated by tangential flow filtration (Pellicon XL Ultracel PLC 5 kd, Millipore) and dialyzed against 20 mM Tris HCI, pH 8.0. Proteins were analyzed on an Agilent 2100 Bioanalyzer with the Protein LabChip. Purified protein solutions were stored at -80 °C.

Protein-specific ELISAs were performed by coating microtiter plates with 100 ng recombinant protein in carbonate buffer, pH 9.6, or with buffer alone. Plates were blocked with 2.5% nonfat dry milk in phosphate-buffered saline containing 0.1% Tween 20 (PBST). One hundred µl of a 1:2000 dilution of serum was applied to duplicate wells for 2 hours, after which plates were washed with PBST and antibody binding was detected by incubation with horseradish peroxidase-conjugated goat-anti swine IgG (heavy + light chains (KPL, Gaithersburg MD) diluted 1:5000 for 1 hour, followed by washing and color development with 100 µl of TMB substrate (KPL). Reactions were stopped with 1 M phosphoric acid and plates were read at 450 nm.

### Body Weights

All pigs were weighed on day 0 (first day of study) and day 49 (end of study). Pigs were weighed on a portable electronic weigh-bar scale system Weigh-Tronix^{TM} model 615XL, (Weigh-Tronix Inc., Fairmont, MN). The scale was calibrated using certified test weights prior to and after each use.

### Clinical Scores

On every day of the study each pig was scored by a veterinarian for respiratory signs, behavior, and coughing on a scale of one to four for each clinical sign. A normal animal was given a score of three, maximum clinical illness was a score nine and a dead animal received a score of 12. Samples from all animals that died in the study were submitted to the Iowa State University Veterinary Diagnostic Laboratory for pathological examination.

### Immunohistological evaluation of lungs

A sample fo lung from each pig was fixed with formalin on the day of necropsy for testing by immunohistochemistry and microscopic examination for lesions compatible with PRRSV. This testing was performed by the Iowa State University Veterinary Diagnostic Lab.

### Statistical Analysis

All data were imported into SAS version 8.02 for data management and analysis. Summary statistics including mean, standard deviation standard error, median and' frequency distributions were generated for all out come.variables as appropriate. Weight, RT-PCR, and Log₁₀ TCID₅₀/ml data were analyzed by one way ANOVA for overall differences among the treatment groups with pairwise testing for differences between treatment groups by Least Significant Difference t test. All tests for differences between groups were designed as two-sided tests. Differences were considered statistically significant at p ≤ 0.05.

Some changes were made to the data to facilitate correlation analyses. The Log₁₀ TCID₅₀/ml values listed as <2.00 were set to 1.0. Negative RT-PCR values were set to 1.0 and all RT-PCR values were normalized by transformation to log base 10 before analysis. Control group results were not included in the correlation analyses. Results for each pig were converted to an approximate area under the curve using trapezoidal rule. Area under the curve was computed for the entire study period, from the first observation to day 15, and from day 15 to the last observation, although only the entire study period is shown in the figures.

### Results

### Virus isolation and Log₁₀ TCID₅₀lml quantification

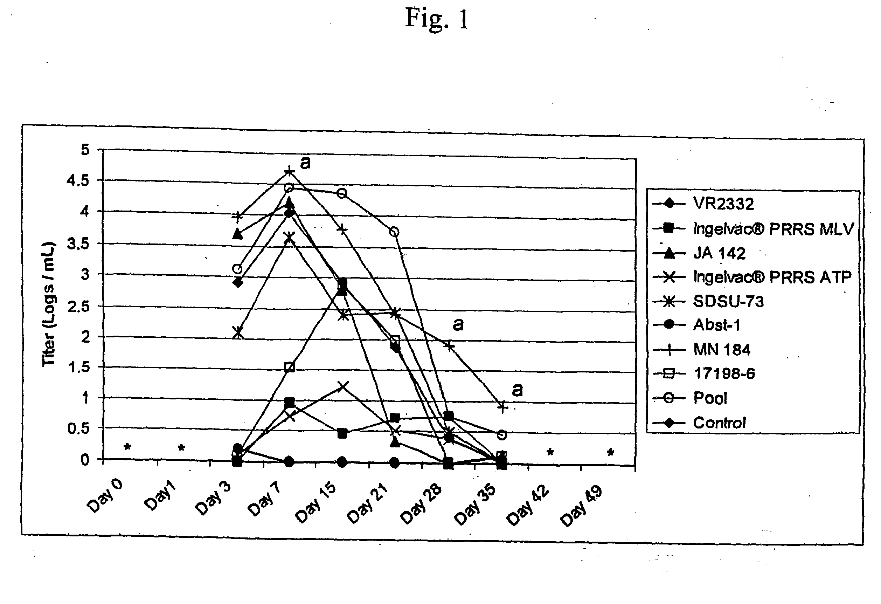

Before exposure on the day of infection no animals tested positive for PRRSV. At 1 day after intranasal infection, only 13 animals in 5 groups tested positive for virus. However, at 3 days after infection all animals that were infected with field isolates, except for isolate 17198-6, were virus positive with meanlog₁₀ TCID₅₀/ml values ranging from 2.1 (SDSU-73) to 3.9 (MN 184). By contrast, animals inoculated with attenuated isolates were uniformly negative by cell culture. These results are provided in Fig. 1. Peak levels of viremia, from 3.6 to 4.6 log₁₀ TCID₅₀/ml were attained on day 7 for four of the five virulent isolates. Isolate 17198-6 peaked on day 15. Titers remained near or above 2 log₁₀ TCID₅₀/ml in all virulent virus groups for 21 days except for JA 142-infected pigs which had titers below that level.

The levels of viremia in the pigs inoculated with attenuated PRRSV isolates were lower than in pigs inoculated with virulent field isolates. The group means of the attenuated isolates peaked at titers over a log lower than the peak of the lowest virulent group titer. The Abst-1 isolate, with the exception of day 3 post inoculation, was never re-isolated. Ingelvac® PRRS MLV viremia fluctuated between 0.5 and 1.0 log₁₀TCID₅₀/ml from days 7 to 28, and Ingelvac® PRRS ATP varied between 0.4 and 1.2 log₁₀CID₅₀/ml from days 7 to 28. Attenuated isolate viruses were not recovered from serum after day 28, and virus was recovered from only two of the virulent field isolate groups, the pool-infected and MN 184-infected pigs through day 35 (also shown in Fig. 1). Nearly all pigs were nonviremic by vims isolation at days 42 and 49.

Overall, the more virulent isolates were observed to replicate faster and to higher titers in pigs than were the attenuated isolates. Pigs infected with the MN 184 isolate, in particular, showed a very rapid increase in virus replication beginning before day 3 and reaching a peak of over 4.5 log₁₀ TCID₅₀/ml on day 7. After peaking, the MN 184 viremia steadily decreased but still maintained a significantly higher titer (t-test, p ≤0.05) than all other isolates on days 28 and 35. A similar trend was observed in all of the remaining virulent groups, namely VR2332, JA 142, SDSU 73, and the pool (see, Fig. 1). Pigs infected with 17198-6 followed the same general trend described for the MN 184 infected group but not as closely.

Groups of pigs administered the attenuated isolates (Ingelvac® PRRS MLV, Ingelvac® PRRS ATP, and Abst-1) followed a different trend. They showed a moderate increase in viral titer beginning after day 3 that reached a peak between days 7 and 15 at a viral titer more than a log less than any of the virulent exposure groups and several orders of magnitude less than the MN 184-infected group. The titers observed in these attenuated exposure groups then declined to zero on or before day 35 (See Fig. 1 ).

When comparing the group means after the second exposure, there is a very steep increase in the viral titers of the attenuated groups, whereas the virulent groups show a smaller increase or no increase at all. For example, Abst-1 had a titer of 3.76 logs seven days after the second exposure, whereas MN 184 had a titer of zero. The other attenuated groups peaked three days after the second exposure. The highest titer observed in any virulent group after the second exposure was 1.32 logs seen three days after exposure in JA 142.

### Virus quantification by Real Time RT-PCR

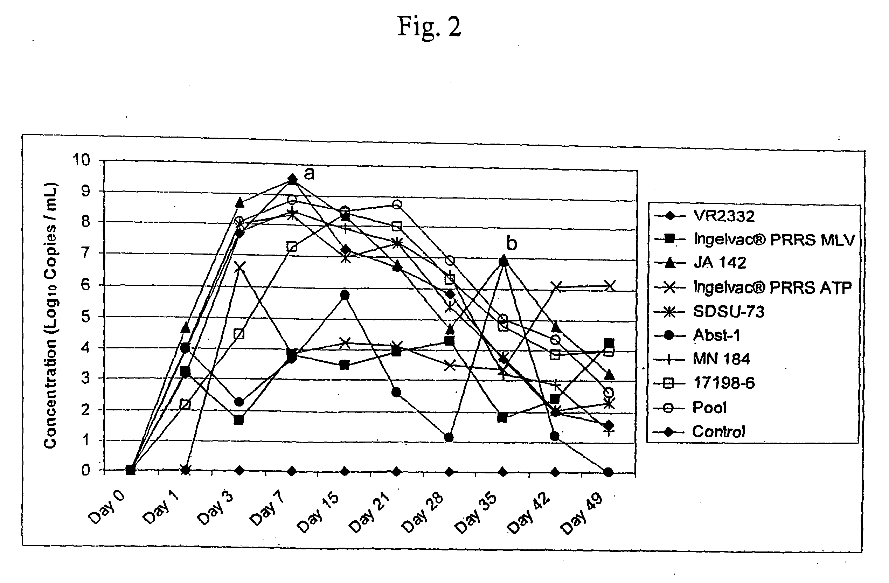

Levels of viremia were also determined by real time RT-PCR since it was possible that growth on CL2621 cells was not the same for all isolates and because RT-PCR might,be a more sensitive measure than growth on cells for viremia. As shown in Fig. 2, virulent exposure groups showed a dramatic increase in average concentration on day 1 and all groups peaked above 8 logs/ml between days 7 and 15. The virulent exposure group concentrations then gradually tapered off through the next several weeks, reaching concentrations below 4 logs/ml by day 49.

The attenuated isolate exposure groups showed a much less dramatic increase in concentration that also began around day 1 and the average group titer never reached or exceeded 7 logs/ml (Fig. 2). The concentrations observed for the attenuated exposure groups were maintained at fluctuating levels showing a wide range in values in the weeks following the exposure. The fluctuations were primarily due to sporadically high values in a single pig. The three attenuated isolate exposure groups all peaked on different days of the study. The Ingelvac® PRRS MLV group peaked at a concentration of 4.31 logs/ml on day 28, the Ingelvac® PRRS ATP group peaked at 6.58 logs/ml on day 3, and the Abst-1 group peaked at 6.85 logs/ml on day 35, which was the highest titer achieved by an attenuated isolate (Fig. 2). Additionally, the average concentration of the virulent isolate groups were observed to be significantly higher (P<0.05) than the average concentration of the attenuated isolate groups on days 3 and 15, but on day 49 the average concentration of the virulent isolate groups was significantly lower (P<0.05) than that of the attenuated isolate groups.

### HerdChek®PRRS ELISA 2XR

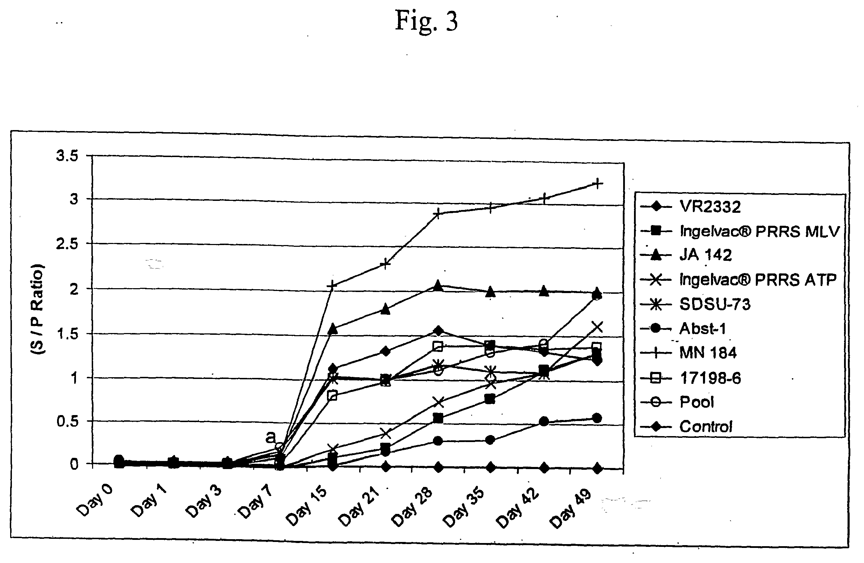

As shown by Fig. 3, the humoral immune response to PRRSV, as measured by HerdChek® PRRS ELISA 2XR S/P ratios, showed that the virulent isolate exposure group averages rose above the 0.4 cutoff for a positive result on day 15. By contrast, the attenuated isolate exposure group averages were negative and all three groups remained below 0.4 until after day 21. The Ingelvac® PRRS MLV and Ingelvac® PRRS ATP groups showed positive results on day 28, but the Abst-1 group did not show an average S/P ratio over 0.4 until day 42.

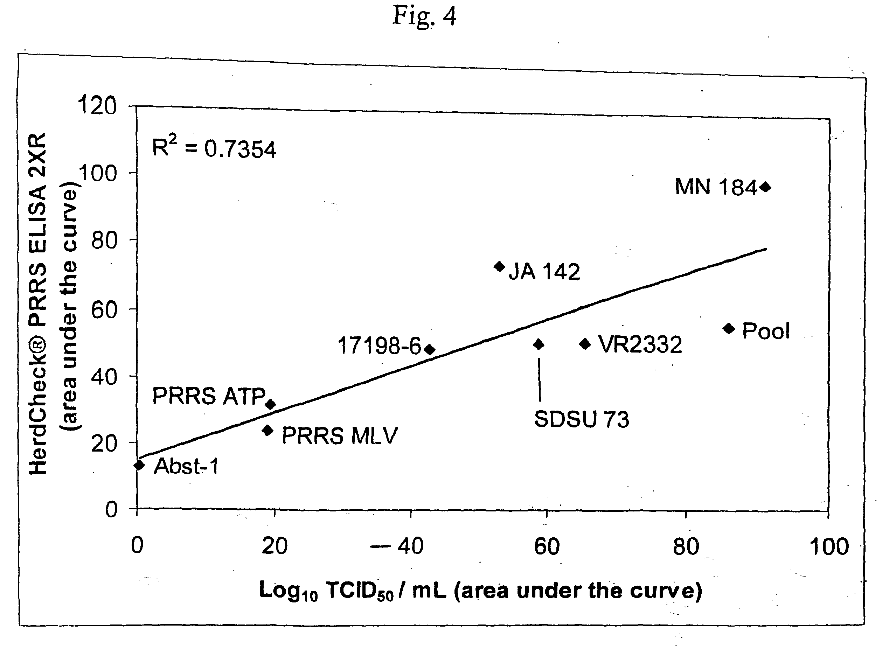

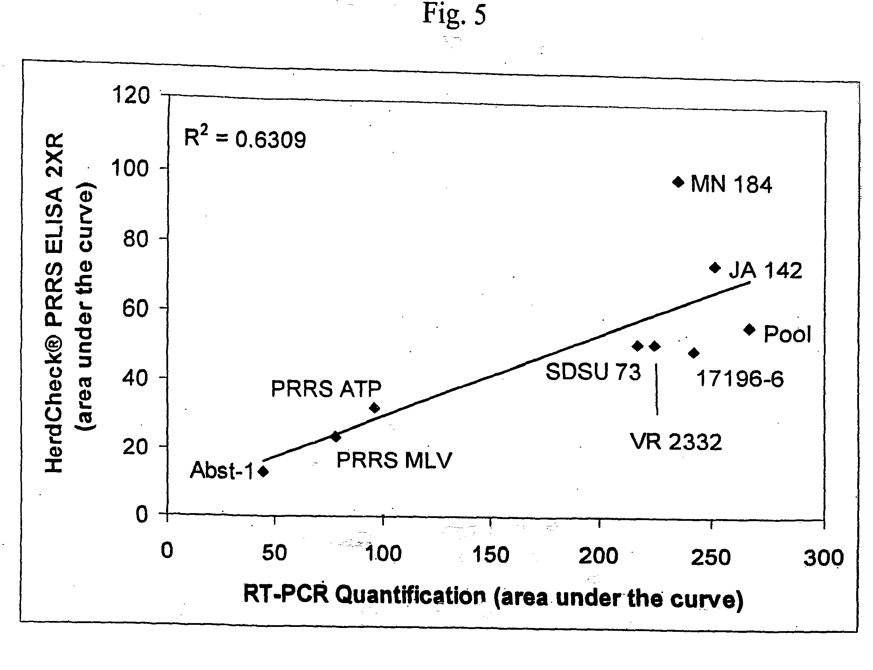

In comparing the humoral response of groups infected with virulent isolates or the pool to groups inoculated with attenuated isolates, it was clear that the kinetics and magnitude of the antibody response was associated with the level of viremia, particularly between 14 and 35 days after infection. This observation is further supported by the correlation between viremia levels and humoral antibody responses determined by paired comparisons of HerdChek® PRRS ELISA 2XR S/P ratios to either virus titration or RT-PCR. Figs. 4 and 5 show that the humoral antibody response is closely associated with viral load over the entire study period with a correlation coefficient *r*=0.858 for virus titration and *r*=0.794 for RT-PCR. These associations were highly significant (p<0.0001 in each case). Moreover, attenuated isolates show low antibody responses and viral loads, whereas virulent isolates show high responses.

### PRRSV Protein-Specific ELISA

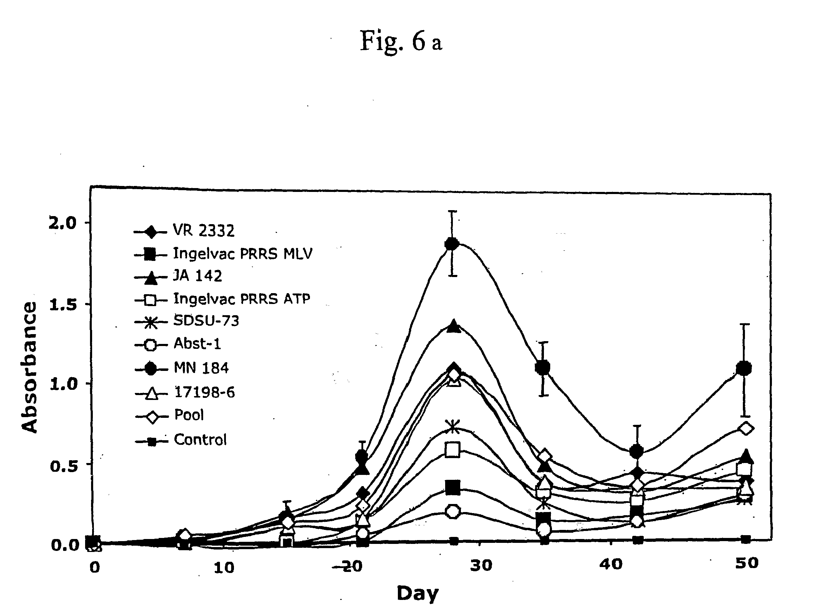

To gain additional insight into the relationship between differences in PRRSV inocula and humoral immune responses, the antibody titers against N, the major structural protein, and nsp 4, an essential but minor nonstructural protease, were determined. Fig. 6a illustrates that the kinetics of the nucleocapsid anti-N IgG response were nearly identical in all groups of.pigs, with a peak titer on day 28 followed by a sharp decline in the next 7-14 days, after which the levels were maintained or rose slightly between days 42 and 49.

The magnitude of the response for each isolate was similar to that found in the HerdChek® PRRS ELISA 2XR results, and consistent with the levels of viremia. The lowest peak titers at day 28 were observed in the groups inoculated with attenuated isolates, and the highest titer was attained in pigs infected with the highly virulent MN 184 isolate. By day 49 the anti-N titer was equivalent in all groups except for MN 184 and the pool, suggesting that the humoral response to MN 184 may be qualitatively different. Additionally, only 5 pigs survived to day 49 in each of these two groups, which is reflected in the increased standard error at day 49 in the MN 184 group.

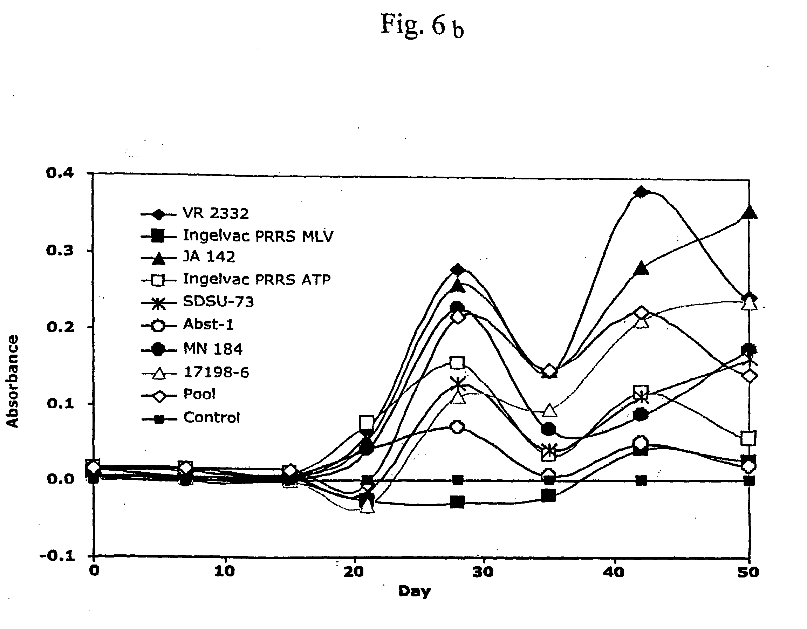

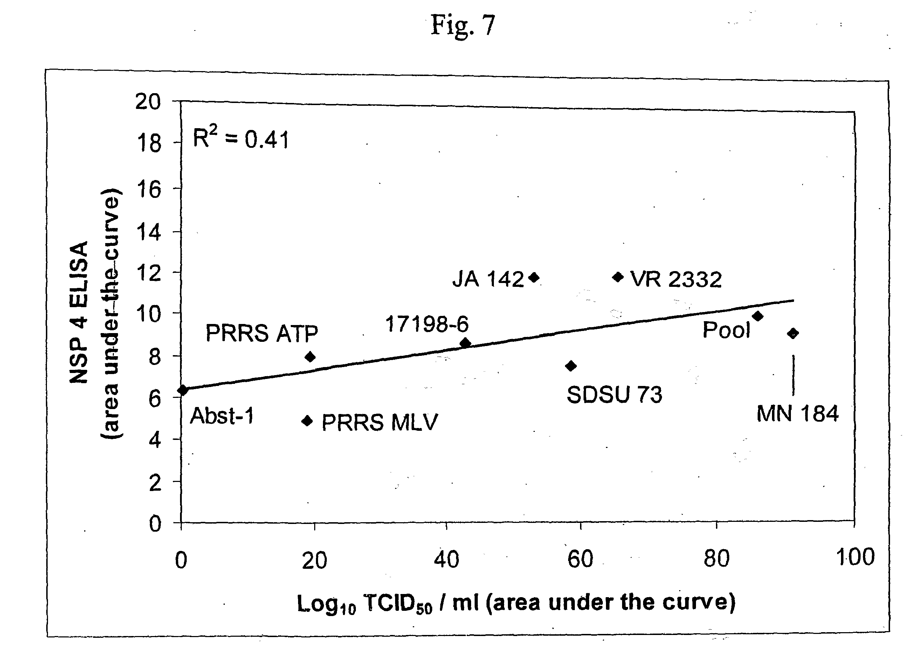

As shown by Fig. 6b, the IgG response to nsp 4 was substantially different than to N. No anti-nsp 4 antibody was detected before day 21, the overall response was much weaker, and no significant response was detected in the groups receiving Ingelvac® PRRS MLV and Abst-1. Moreover, the magnitude of the anti-nsp 4 response was not associated with level of viremia. The responses to VR 2332, JA 142, MN 184, and the pool were all equivalent, with a peak at day 28, followed by a decline at day 35, then rising again at day 42, whereas the magnitude, time course and duration of viremia varied among these four groups. Fig. 7 illustrates that when examining the repeated measures analysis, data for the nsp 4 ELISA compared to the Log₁₀ TCID₅₀/ml data, it can be seen that there is no correlation between level of viremia and nsp 4 humoral antibody response. There was also very little secondary response following the second exposure on day 49 to isolate MN 184.

### Body weight

There was no significant difference in the mean weight of any of the groups on day 0 of the experiment (P = 0.099). On day 49 pigs inoculated with the attenuated isolate Abst-1 had the highest mean weight, which was significantly higher then all other groups except for the control group (Table 3). Also, on day 49, the mean weights of all the virulent isolate exposure groups except for the 17198-6 group were significantly lower than the control group (Table 3). The mean weights of the attenuated isolate exposure groups Ingelvac® PRRS MLV and Ingelvac® PRRS ATP and the control group were statistically equivalent (Table 3).

**Table 3. Average Body Weights.**

| Isolate | Day 0 | Day 49 |
|---|---|---|
| VR 2332 | 6.38¹ | 33.5^{b} |
| Ingelvac® PRRS MLV | 6.56 | 34.6* |
| JA 142 | 6.42 | 32.7^{b} |
| lngelvac® PRRS ATP | 6.24 | 35.0* |
| SDSU-73 | 6.59 | 32.9^{b} |
| Abst-1 | 6.69 | 39.4^{a} |
| MN 184 | 6.73 | 23.7^{c} |
| 17198-6 | 6.36 | 34.5* |
| Pool** | 6.51 | 23.0^{c} |
| Control | 6.48 | 38.4* |

| | | |
|---|---|---|
| ¹ Weights are in kg. There were no significant differences in mean wt at day 0. * Indicates statistically equivalent weights among these groups on day 49. ** Pool was a mixture containing all eight isolates. ^{a} Significantly greater than all groups except the Control group (p≤ 0.05). ^{b} Significantly less than the Control group. ^{c} Significantly less than all other groups. | | |

### Clinical Scores

Increases in average clinical scores were observed in only four of the virulent exposure groups: JA 142, SDSU 73, MN 184, and the Pool. These higher scores were maintained throughout the study while the remaining groups, both virulent and attenuated exposures, had essentially normal clinical scores for the duration of the study. The only major cause of changes in the average clinical scores observed in this study occurred when one or more animals died in the associated treatment group (Table 4).

**Table 4. Mortality of Pigs after Exposure**

| Group | Isolate | Mortality | Day(s) of Death(s) |
|---|---|---|---|
| 1 | VR 2332 | 0/10 | N/A |
| 2 | Ingelyac® PRRS MLV | 0/10 | N/A |
| 3 | JA 142 | 1/10 = 10% | 17 |
| 4 | lngelvac® PRRS ATP | 0/10 | N/A |
| 5 | SDSU 73 | 2/10 = 20% | 9,23 |
| 6 | Abst-1 | 0/10 | N/A |
| 7 | MN 184 | 5/10 = 50% | 14, 14, 17, 23, 41 |
| 8 | 17198-6 | 0/10 | N/A |
| 9 | Pool** | 5/10 = 50% | 12, 16, 17, 21, 21 |
| 10 | Controls | 2/10* | 41,48 |
| | | | |
| Attenuated PRRSV | | 0/30 = 0% | |
| Virulent PRRSV | | 13/60 = 22% | |

| | | | |
|---|---|---|---|
| All deaths in treatment groups were attributed to moderate or severe non-suppurative interstitial pneumonia due to PRRSV with secondary bacterial infection. * Deaths attributed to bacterial pneumonia with no PRRS involvement. ** Pool was a mixture containing all eight isolates. | | | |

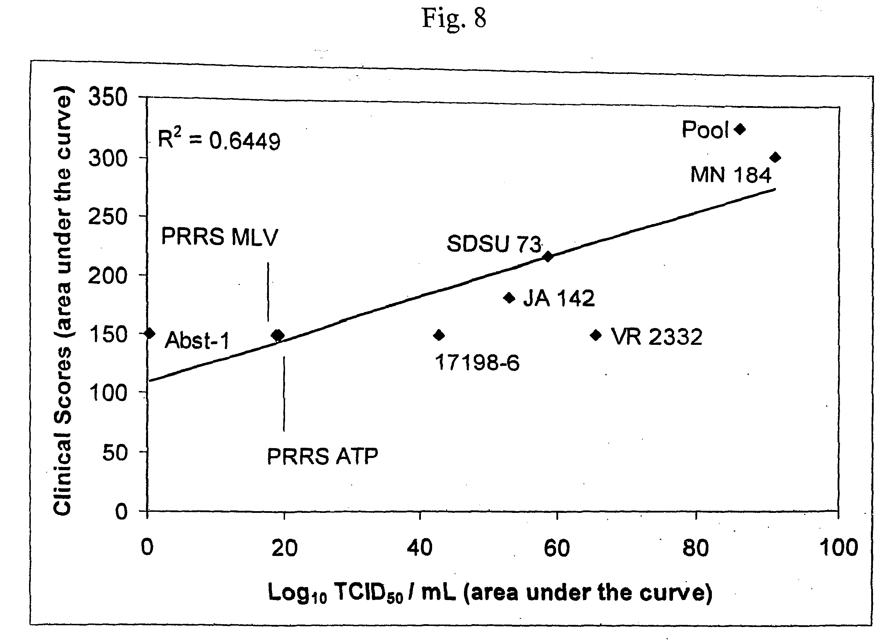

The severity of clinical disease was highly associated with viral load (p<0.001 for virus titration). As shown in Fig. 8, the clinical scores were highest for the groups infected with MN 184 and the Pool. Fifty percent of the pigs in each group died, and virus titration indicated that the level of infection was substantially higher than for all other groups. The differences in viral load as determined by RT-PCR were less marked (data not shown) and the correlation of clinical signs with viral load by RT-PCR was less than with virus titration (*r*=0.556 versus *r*=0.803, respectively). The clinical scores in group 10 (Control) increased after the death of two pigs from bacterial pneumonia. Both pigs were shown to be PRRSV negative by immunohistochemical staining of lung tissue, negative virus isolation and real-time PCR analyses, and the complete lack of seroconversion by HerdChek® PRRS ELISA 2XR or protein-specific ELISA. Later findings indicated that various bacterial pathogens were present in animals that died unexpectedly during the study these deaths were likely attributed to secondary bacterial infection (Table 5).

**Table 5. Cause of Mortality after Exposure**

| Pig # | Group | Cause of Death | Day of Death |
|---|---|---|---|
| 993 | JA 142 | PRRS & *Streptococcus suis* | 17 |
| 948 | Neg Control | *Arcanobacterium pyogenes & Pasteurella multocida* | 41 |
| 983 | Neg Control | *A. pyogenes & P. multocida* | 48 |
| 922 | SDSU 73 | PRRS and bacterial pneumonia* | 9 |
| 918 | SDSU 73 | PRRS and *Escherichia coli* | 23 |
| 973 | MN 184 | PRRS and *Actinobacillus suis* | 14 |
| 992 | MN 184 | PRRS and *A. suis* | 14 |
| 980 | MN 184 | PRRS and *E.coli* | 17 |
| 971 | MN 184 | PRRS and *E.coli* | 23 |
| 958 | MN 184 | PRRS and *E.coli* | 41 |
| 976 | Pool | PRRS and *A. suis* | 12 |
| 970 | Pool | PRRS and V *S*. *suis* | 16 |
| 972 | Pool | PRRS and *S*. *suis* | 17 |
| 995 | Pool | PRRS and *S*. *suis* | 21 |
| 969 | Pool | PRRS and *A. pyogenes* | 21 |

| | | | |
|---|---|---|---|
| * The diagnostic report indicated "bacteria] pneumonia" with no specific agent listed. | | | |

### Immunohistological evaluation of lungs

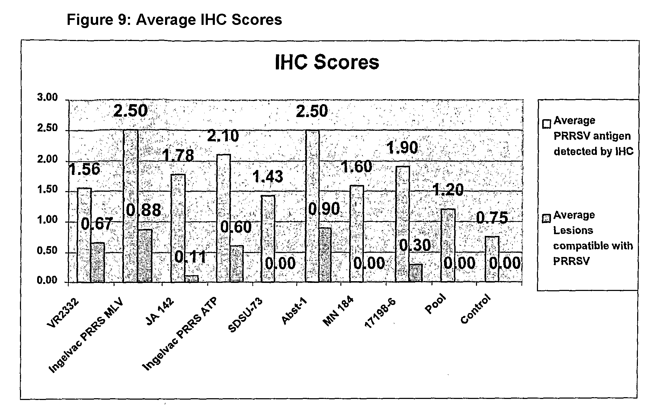

Although the mean IHC scores and PRRSV lesions observed for individual groups were not significantly different, a significant difference was seen, when comparing IHC scores according to virulence. For mean IHC scores and PRRSC lesions see Fig. 9 and Table 6.

**Table 6: Mean IHC Scores Based on Virulence.**

| **Variable** | **AVlR Mean** | **VIR Mean** | **ANOVA p Value** |
|---|---|---|---|
| **IHC** | 2.36 | 1.49 | **<0.0001*** |

| | | | |
|---|---|---|---|
| * = Significant at p ≤ 0.05 | | | |

### Discussion

One objective of this example was to examine various PRRSV isolates with known levels of virulence to determine if there was a relationship with *in vivo* replication that could be used to predict the virulence of PRRSV isolates without the necessity of performing controlled challenge experiments. Additionally, it was of interest to determine the relationship between isolate virulence, levels of viremia, and the humoral antibody response. Finally, it may be of interest to develop vaccines against the PRRSV isolates that are found to be virulent using the methods of the present invention. It would be a goal to have such vaccines provide some degree of protection against other virulent isolates; however, such cross-effectiveness may not be universal for all PRRSV isolates and further testing would be required. However, it is clear that the present invention provides an effective tool for identifying prime candidates for vaccine development.

In order to test PRRSV isolates under the same conditions it was necessary to use dosages of licensed vaccines that were below the minimum immunizing dose established with the USDA and that were not representative of a commercial dose. Also, the intranasal route of administration of the MLV vaccines used in the study was not in accordance with the USDA label and was only used to mimic a more natural exposure. The typical commercial dose of the modified live PRRS vaccines (Ingelvac PRRS and Ingelvac PRRS ATP) is much higher than what was used in this experimental trial. These experimentally low doses of modified live PRRS vaccine do not represent the actual product dosage and form used in the field and readily explains the reported serological response. Using a commercial dose of vaccine, serological titers as measured via the IDEXX assay would be detectable by day 14. In this trial using titers of approximately 3 logs, this serological response was delayed and lower. This was to be expected, but was done to insure consistency of titer administration between groups and to facilitate the analysis and comparison between virulent and attenuated isolates. Although not specifically addressed in this Example, the effect of dose is likely much more significant for an attenuated or less virulent virus than it is for a virulent field virus that can quickly grow in and be recovered at over 4 logs/ml in pig serum within 3-7 days of exposure. The higher recommended intramuscular commercial dose gives HerdChek® PRRS ELISA 2XR S/P ratios above the 0.4 cutoff by 14 days post vaccination which is one-half the amount of time observed for the doses used in this study (Roof et al., 2003). The nominal dose used in this study, 2 x 10³ TCID₅₀ per animal, caused 50% mortality in groups that received isolate MN 184, and anti-nucleocapsid responses in all groups. Higher doses were not tested since excessive mortality in groups challenged with highly virulent isolates would have compromised the study objectives. In addition, previous studies had shown no difference in clinical signs and viremia in young pigs inoculated with PRRSV isolate VR2332 at doses of 10^{2.2}, 10^{3.2} and 10^{4.2} TCID₅₀ per animal.

Both the Log₁₀ TCID₅₀/ml and real time RT-PCR results showed that the viremia levels vary significantly among groups following PRRSV exposure. This indicates that the growth rate of PRRSV in pigs is a phenotypic characteristic of the virus independent of possible variation in pig susceptibility to infection. ln addition, attenuation of PRRSV by adaptation to growth on CL2621 cells reduced not only its ability to grow in pigs, but altered the kinetics of viral replication so that peak viremia occurred at later times. A similar observation was also made by Chang et al. (2002), who showed that even a limited period of cell culture passage of the moderately virulent PRRSV isolate VR 2332 reduced viral growth in pigs and delayed significantly the time to peak viremia. However, a delayed time to peak viremia is not diagnostic for *in vitro* cell culture passage or for attenuation, since the highly virulent isolate 17198-6 also showed a delayed time to peak viremia.

Overall, virulent isolates showed substantially higher viremia levels in serum than did attenuated isolates at equivalent doses of inoculation. For example, the highest observed virus liter in any of the attenuated isolate exposure groups was 1.22 logs on day 15 in pigs given Ingelvac® PRRS ATP, whereas the lowest titer of any virulent group on day 15 was 2.40 logs in the SDSU 73 group. The peak of viremia at days 3-7 and the levels of virus detected (all >3.5 logs/ml) was highly consistent among virulent PRRSV isolates, though MN 184 was significantly greater in its magnitude and duration, with virus titers still present on days 28 and 35. This supports the concept that highly virulent PRRSV isolates replicate to a substantially higher titer *in vivo* than do attenuated or lowly virulent isolates, but they do not establish a direct quantitative relationship between level of virulence and level or rate of *in vivo* growth among wild-type PRRSV (Haynes et al. 1997). The groups showed a wide variety of protection from the second exposure with the MN 184 isolate. For example, there was no virus detected in the MN 184 or pool group, both of which received the MN 184 isolate initially. It can be seen that Abst-1 confer very little protection following exposure to MN 184. These observations further solidify the concept that homologous exposure confers more protection than does heterologous exposure. The homologous virulent exposure was more protective and the heterologous virulent exposure was less protective, but still more protective than the heterologous attenuated exposure.

The real time RT-PCR results were statistically very similar to the Log₁₀ TCID₅₀/ml results, indicating that both methods measure relative levels of infectious virus among groups. The Pearson correlation coefficient between the RT-PCR and Log₁₀ TCID₅₀/ml day 7 data was 0.89 and for the average real time RT-PCR and Log₁₀ TCID₅₀/ml results was 0.88. The concentration values determined by real time RT-PCR may have been several orders of magnitude higher than logo₁₀ TCID₅₀/ml values for several reasons, including differences between the frequency of viral particles containing the target amplicon and particles that are fully infectious on CL2621 cells, and the presence of neutralizing antibodies that could lower infectivity (Dianzani et al., 2002). However, neutralizing antibody is unlikely to account for the difference, because it was observed at all time points, including times before which an, anti-PRRSV antibody response had been produced.

The copies/ml values determined by real-time PCR were higher than the infectious titer values measured in cell culture by TCID₅₀/ml. This is because a standard curve based on the copies of genome of the virus is routinely used for quantitative PCR which directly amplifies a genomic sequence of the virus rather than known infectious virions. Biologic assays such as cell culture do measure the presence of infectivity, however, they may not count all of the infectious particles present in a preparation. Factors that could affect the infectious titer such as cell culture conditions and *in vivo* antibodies, which may neutralize virus, have been observed in other studies, underestimating the amount of infectious virus measured in TCID₅₀/ml in sera. Alternatively, some non-infectious or replication-defective virus may be present which would be reflected by higher copy numbers.

In general, the ELISA observations support the concept that the magnitude of the humoral immune response is related to the level of viral replication during acute infection. The trend indicated in Figs. 4 and 5 illustrates this relationship. A slower and less intense humoral immune response was triggered by the cell-culture attenuated virus isolates, whereas a faster and more intense humoral immune response was triggered by the virulent isolates. In addition these observations also demonstrate that at least two factors, isolate type and infectious dose, impact relative S/P ratio values in the HerdChek® PRRS ELISA 2XR. Although the ELISA results shown in Fig. 3 indicate a clear positive or negative average group response, it is important to note the variability among individual animals. Some pigs within attenuated virus groups were positive before day 21, and some pigs in the virulent groups remained negative up to day 21.

Analysis of specific antibody responses to N and nsp 4 show that immune responses to PRRSV vary in intensity independently of the inoculating isolate. Antibody responses to the N protein in animals that were inoculated with the highly virulent isolates MN 184 and JA 142 showed a trend similar to that of all the isolates but to a higher magnitude. Pigs inoculated with MN 184 and JA142 also had the highest viral titers, as shown in Figs. 1 and 2. This indicates that the level of humoral immune response may be related to the viral load in acute infection as measured by viral titer. Interestingly, the time course of response was the same in all groups, even though the time to peak titer was delayed for highly virulent isolate 17198-6 and the attenuated isolates. The nsp 4 antibody response, by contrast, was low at all of the time points and for all of the isolates, both attenuated and virulent. The time course of anti-nsp 4 response was equivalent in all of the groups despite differences in the time to peak viral load among groups, as observed for the anti-N antibody response. All pigs had low anti-nsp 4 responses as shown in Fig. 7.

These observations indicate that some of the PRRSV proteins elicit a more robust response from the host immune system regardless of exposure isolate virulence. However, the observations also indicate that the magnitude of the immune response to the more immunogenic proteins is likely related to the virulence of the exposure isolate, or the ability of the isolate to replicate *in vivo.* It also is possible that differences in antibody response might be due simply to genetic differences among isolates that result in differences in antigenic reactivity such that antibodies directed against N and nsp 4 of other isolates do not react or react poorly to the recombinant proteins expressed from isolate VR2332 that were used to coat the ELISA plates. However, several lines of evidence suggest that the observed differences in antibody levels reflect immunologically relevant responses. Isolate MN 184 shows the greatest genetic difference from VR2332, as determined by ORF 5 comparisons, yet has the highest anti-N antibody response. Kapur et al. (1996) showed previously that relative differences among PRRSV isolates in one open reading frame are also present in other open reading frames. Additionally, individual proteins contain conserved and nonconserved regions (e.g. Kapur et al., 1996) and extensive immunogenic reactivity may be directed toward the conserved epitopes (Ostrowski et al., 2002). Nevertheless, ELISA results based on antibody reactions with purified PRRSV proteins may be affected by genetic and antigenic variation, and these effects must be considered. Refolding of recombinant proteins was performed, but no differences were observed between ELISA plates coated with nonrefolded or refolded proteins.

It was noted that at approximately 4 to 5 weeks after inoculation, a relatively large decrease in the antibody response to both the N and nsp 4 proteins occurred. A similar peak of 1 to 2 weeks followed by a decline of antibody reactivity was previously noted by Foss et al. (2002) for GP5, the major envelope glycoprotein. Taken together, these observations suggest that the response to individual viral proteins likely does not represent the full picture of the pig's immune response to PRRSV since the humoral immune response as measured by the HerdChekⓇ PRRS ELISA 2XR does not show a similar transient peak of antibody reactivity.

Reduced growth and mortality were the key correlates of virulence and viral *in vivo* growth rate. The lower mean weight observed in the virulent isolate exposure groups most likely reflected a difference in the ability of a PRRSV isolate to replicate *in vivo* and induce a more severe illness in the pig. These observations are consistent with previously reported data that PRRSV infection may cause anorexia with a 25 to 40 percent reduction in daily weight gain (Thacker, 2003). The clinical scores of animals exposed to the virulent isolates showed rapid increases shortly after the inoculation, whereas there was virtually no change in the scores of the attenuated virus exposure animals. This increase in clinical signs was reflected in the observed death rates of 50%, 20%, and 10% in the virulent exposure groups receiving PRRS isolates MN 184, SDSU 73, and JA 142, respectively. In contrast, the attenuated exposure groups incurred no deaths. The relationship between rapid viral growth and viral pathogenesis under the same conditions of viral exposure were most evident in comparing the groups exposed to MN 184 and Abst-1. The inoculation titers were virtually the same, 4.10 logs/ml and 4.18 logs/ml, respectively, and yet, as indicated in Fig. 8, there were remarkable differences in the way the two isolates affected pigs. The Abst-1 isolate was nearly inert, it hardly replicated *in vivo* and caused no clinical signs. By contrast, the MN 184 isolate replicated to extremely high titers *in vivo* and caused severe clinical signs, resulting in the death of 50% of exposed animals. Also notable, the group of pigs exposed to the pool of all virus isolates showed about the same virological, clinical, and immunological responses as pigs exposed to MN 184. This finding indicates that the most rapidly replicating virus in a mixed infection is likely to outcompete other isolates so that the net result is essentially the same as an infection with the single isolate having the highest growth potential.

The notable *in vivo* differences between virulent and attenuated PRRSV isolates shed light on the relationship between the virulence of an isolate and its *in vivo* growth and replication. When administered at equivalent doses in pigs, the more virulent isolates show Log₁₀ TCID₅₀/ml titers and RT-PCR concentrations that are exponentially higher than the attenuated isolates. The virulent isolates induce a more rapid and intense humoral immune response. The virulent isolates negatively affect weight gain and induce higher death rates and more severe clinical signs as compared to the attenuated isolates.

In conclusion, the example and tests in the present application indicate that attenuated and virulent PRRSV isolates induce remarkably different clinical signs, as well as immune responses that differ in intensity. These differences are attributed to the ability of the virus to replicate *in vivo,* a phenotypic characteristic that can be measured quantitatively in serum samples and may be developed for predicting the virulence of PRRSV isolates.

### EXAMPLE 2

This example provides several methods of attenuating PRRS isolates as well as their inclusion in immunogenic compositions.

### Materials and Methods:

A vaccine preparation is formulated incorporating a modified or attenuated live virus for the immunization of swine from infection by PRRS. The PRRSV virus for the vaccine preparation is propagated in the MA-104 continuous cell line, preferably ATCC No. CL2621. The cell line is grown in flasks containing MEM, to which is added 10% fetal calf serum. The pH of the media is adjusted to approximately 7.2, and incubated at approximately 37° C. The cells are then inoculated with the virus by adding about 1 ml of a frozen inoculum to the fluid media. The virus is allowed to absorb onto the cells for 24 hours. At this time, the growth media is changed to a maintenance media consisting of MEM to which has been added 4% fetal calf serum, pH 7.6. The environment is preferably between 35°-37° C. The virus is allowed to grow until 50% of the MA-104 cell sheet is destroyed by the virus. The sample is then frozen down, and prepared for passage onto another flask of MA-104 cells. This process is continued through 25 passages of the virus in the cell line. The virus is then propagated another 12 times at about 31° C. as opposed to 35°-37° C., using the same techniques as described above. The 12the passage is frozen down in small aliquots and designated as the Master Seed Virus.

The following sets forth the details of the preferred method.

### I. Media:

a. Eagles Minimum Essential Medium (MEM) from JRH Biosciences, #200-2041
b. Fetal calf serum (FCS) from JRH biosciences
c. Growth media for cell planting-MEM+10% Fetal Calf Serum
d. Maintenance media-MEM 4% Fetal Calf Serum
e. Trypsin-Versene IX
f. Sodium Bicarbonate 5% or saturated

### II. Tissue Culture:

Cell line used: MA-104 (African Green Monkey Kidney cells kept within 20 passage levels (passage 58-78)

### III. Equipment:

75cm tissue culture flasks
Incubator set at 35°-37° C.
Incubator set at 3° C.
Centrifuge

### IV. Method Used to Attenuate PRRSV Virus Grown at 25°-37° C.

### A. Preparation of Tissue Culture Stock:

5 to 7 day old MA-104 75cm² stock bottles are split 1:4 in the following manner:
a. Pour off all media (50ml per bottle).
b. Remove the cell sheet using 10ml of Trypsin-Versene by incubation at 37° C. for 5-10 minutes.
c. Remove cells from bottle and centrifuge 270xg for 5-10 minutes.
d. Decant supernatant and resuspend the cells in 5- 10ml of growth media (MEM 10% FCS).
e. Put all cells into 200ml of MEM 10% FCS which is then dispensed into four 75cm bottles, 50ml per bottle for a 1:4 split. Bottles are then kept at 35°-37° C. until needed (can be done also without CO₂).
f. 3 or 4 day old bottles which have formed a full cell sheet are now ready for use.
g. Adjust pH of 50ml of media in flask to 7.2 pH and then add 1ml of virus into media and put flask at 35°-37° C. (can be done also without CO₂).
h. After 24 hours, media is discarded and flask is refed with 50ml of MEM 4% FCS 7.6 pH and put back at 35°-37° C.
i. 24 hours after this fluid change, CPE should be showing and, when 50-60% holes are present in cell sheet, freeze down.
j. Thaw out above bottle and take 1 ml of this fluid and pass into new 75cm bottle as listed above to make next passage of virus.

This above procedure is carried out for a total of 25 times, grown at 35°-37° C.

Method Used to Attenuate PRRSV Virus Grown at 31° C. Virus passed 25 times at 35°-37° C. is used to make passage 1 for virus grown at 31° C.

### A. Preparation of Tissue Culture Stock:

5 to 7 days old MA-104 75cm² stock bottles are split 1:4 in the following manner:
a. Pour off all media (50ml per bottle).
b. Remove the cell sheet using 10ml of Trypsin-Versene by incubation at 37° C. for 5-10 minutes.
c. Remove cells from bottle and centrifuge 270xg for 5-10 minutes.
d. Decant superuatant and resuspend the cells in 5-10ml of growth media (MEM 10% FCS).
e. Put all cells into 200ml of MEM 10% FCS which is then dispensed into four 75cm bottles, 50ml per bottle for 1:4 split. Bottles are then kept at 35°-37° C. until needed (can be done also without CO₂).
f. 3 or 4 day old bottles which have formed a full cell sheet are now ready for use.
g. Adjust pH of 50ml of media in flask to 7.2 pH and then add 1ml of virus into media and put flask at 31° C. (can be done also without CO₂).
h. After 24 hours, media is discarded and flask is refed with 50ml of MEM 4% FCS 7.6 pH and put back at 31°C.
i. 24 hours after this fluid change, CPE should be showing and, when 50-60% holes are present in cell sheets, freeze down.
j. Thaw out above bottle and take 1ml of this fluid to make next passage of SIRS VR-2332 virus.

This above procedure is carried out for a total of 12 times at 31°C. The 12th passage of 31°C. grown vims is designated Master Seed Virus for vaccine production.

### Attenuation of PRRS virus by Gene Deletion

One skilled in the art may attenuate PRRSV through the conventional method of gene deletion. Generally, this method involves the deletion of a gene which codes for the virulent phenotypes of the virus. Such a method may be adapted from of such references as Elbers et al. (U.S. Pat. App. No. 20020012670) or Schall et al (U.S. Pat. No. 6,740,324), the teachings and content of which are incorporated by reference herein.

### Attenuation of PRRS virus by Temperature-Sensitive Mutation

One skilled in the art may also attenuate PRRSV through the conventional method of temperature sensitive mutation. Generally, this method involves causing a mutation that limits the temperature range in which the virus is active. For example, the method of Skiadopoulos et al., "Identification of Mutations Contributing to the Temperature-Sensitive, Cold-Adapted, and Attenuation Phenotypes of the Live-Attenuated Cold-Passage 45 (cp45) Human Parainfluenza Virus 3 Candidate Vaccine", 73 No. 2 Journal of Virology 1374, (February 1999) may be adapted for use with PRRSV. The teachings and content of Skiadopoulos et al. are incorporated by reference herein.

### Attenuation of PRRS virus by Generation of an Infectious Clone

One skilled in the art may also attenuate PRRSV through the conventional method of generating an infectious clone. This method generally involves the creation of a full-length cDNA clone of the desired viral genotype, said clone also being virulent in the target animals. For example, the method of Nielson et al., "Generation of an Infections Clone of VR-2332, a Highly Virulent North American-Type Isolate of Procine Reproductive and Respiratory Syndrome Virus", 77 Journal of Virology 3702 (Mar. 2003), may be adapted for an attenuated version of PRRSV. The teachings and content of Nielson et al. are incorporated by reference herein.

### Attenuation of PRRS virus by Gene Insertion

One skilled in the art may also attenuate PRRSV through the conventional method of gene insertion. This method generally involves the insertion of a gene within the virus which inhibits the pathogenic phenotypes of said virus. For example, the method of Schall et al. (U.S. Pat. No. 6,740,324) may be adapted for such an attenuated version of PRRSV. The teachings and content of Schall et al. are incorporated by reference herein.

### Attenuation of PRRS virus by Substitution

One skilled in the art may also attenuate PRRSV through the conventional methods of codon and base-pair substitution. Generally, codon or base-pair substitution involves substituting a codon or base-pair with a different codon or base-pair such that the viral gene now codes for a protein which limits the pathogenicity of the virus. For example, the method of codon substitution of McAuliffe et al., "Codon Substitution Mutuations at Two Positions in the L Polymerase Protein of Human Parainfluenza Virus Type 1 Yield Viruses with a Spectrum of Attenuation In Vivo and Increased Phenotypic Stability In Vitro", 78 Journal of Virology 2029 (February 2004) may be adapted to create a gene substitution method for attenuating PRRSV. Also, Hurrelbrink and McMinn, "Attenuation of Murray Valley Encephalitis Virus by Site-Directed Mutagenesis of the Hinge and Putative Receptor-Binding Regions of the Envelope Protein", 75 Journal of Virology 7692 (August 2001) and Elbers et al. (US Pat. App. No. 20020012670) provide methods of base-pair substitution that may be adapted to create an attenuated PRRSV isolate. The teachings and content of McAuliffe et al., Hurrelbrink and McMinn, and Elbers et al. are incorporated by reference herein.

### Attenuation of PRRS virus by Use of Chimeric PRRS Constructs

One skilled in the art may also attenuate PRRSV through the conventional method of chimeric constructs. Generally, a chimeric construct is one that contains genes from different species in order to induce an antigenic response in a host cell. For example, such a construct may be created by adapting the method of Harris et al. (U.S. Pat. App. No. 20040157307) to create a chimeric construct with PRRS genes.

### Incorporation of attenuated PRRS isolates into immunogenic compositions:

After new isolates are attenuated, preferably by one of the methods noted above, the attenuated isolates can be incorporated into compositions effective at providing an immune response against virulent isolates of PRRS. In preferred forms, such compositions will provide an animal receiving an effective amount of the composition, protective immunity agains PRRS infection. Such protective immunity will reduce the severity of clinical signs of PRRS infection in vaccinated animals that are subsequently exposed to or infected by a virulent PRRS isolate. Preferably, such clinical signs will be prevented by the administration of an effective amount of the immunogenic composition or vaccine. In some forms, the attenuated PRRS isolates will be used to produce a modified live vims vaccine, in which the attenuated isolates are used in their live state in the immunogenic compositions. In other forms, the attenuated isolates will be killed or inactivated prior to their incorporation into immunogenic compositions.

### References

The teachings and content of each of the following references are incorporated by reference herein.
Albina, E., Piriou, L., Hutet, E., Cariolet, R., L'Hospitalier, R., 1998. Immune Responses in Pigs Infected with Porcine Reproductive and Respiratory Syndrome Virus (PRRSV). Vet. Immunol. Immunopathol. 61, 49-66.
Andreyev, V. G., Wesley, R. D., Mengeling, W. L., Vorwald, A. C., Lager, K. M., 1997. Genetic Variation and Phylogenetic Relationships of 22 Porcine Reproductive and Respiratory Syndrome Virus (PRRSV) Field Strains Based on Sequence Analysis of Open Reading Frame 5. Arch. Virol. 142, 993-1001.
Cavanagh, D.,1997. Nidovirales: a New Order Comprising Coronaviridae and Arteriviridae. Arch. Virol. 142, 629-633.
Chang, C. C., Yoon, K. J., Zimmerman, J. J., Harmon, K. M., Dixon, P. M., Dvorak C. M. T., Murtaugh, M. P., 2002. Evolution of Porcine Reproductive and Respiratory Syndrome Virus During Sequential Passages in Pigs. J. Virology. 76, 4750-4763.
Grebennikova, T. V., Clouser, D. F., Vorwald, A. C., Musienko, M. I., Mengeling, W. L., Lager, K. M., Wesley, R. D., Biketov, S. F., Zaberezhny, A. D., Aliper, T. I., Nepoklonov, E. A., 2004. Genomic Characterization of Virulent, Attenuated, and Revertant Passages of North American Porcine Reproductive and Respiratory Syndrome Virus Strain. Virology. 321, 383-390.
Dianzani F., G. Anelli, E.Riva, O. Turriziani, L. Antonelli, S. Tyring, D. Carrasco, H. Lee, D. Nguyen, J. Pan, J. Poast, M. Cloyd, S. Baron. 2002. Is Human Immunodeficiency Virus Rna Load Composed Of Neutralized Immune Complexes? Journal of Infectious Diseases: 185:1051-1054.
Foss, D.L., Zilliox, M.J., Meier, W., Zuckermann, F., Murtaugh, M.P. 2002. Danger Signals Increase the Immune Response to Porcine Reproductive and Respiratory Syndrome Virus. Virus Res. 15, 557-566.
Halbur, P. G., Pallares, F. J., Rathje, J. A., Evans, R., Hagemoser, W. A., Paul, P. S., Meng, X. J., 2002. Effects of Different Us Isolates of Porcine Reproductive and Respiratory Syndrome Virus (PRRSV) on Blood and Bone Marrow Parameters of Experimentally Infected Pigs. Vet Rec. 21, 344-348.
Halbur, P. G., Paul, P. S., Meng, X. J., Lum, M. A., Andrews, J. J., Rathje, J. A., 1996. Comparative Pathogenicity of Nine Us Porcine Reproductive and Respiratory Syndrome Virus (PRRSV) Isolates in a Five-week-old Cesarean-derived, Colostrums-deprived Pig Model. J. Vet. Diagn. Invest. 8, 11-20*.*
Haynes, J. S., Halbur, P. G., Sirinarumitr, T., Paul, P. S., Meng, X. J., Huffman, E. L., 1997. Temporal and Morphologic Characterization of the Distribution of Porcine Reproductive and Respiratory Syndrome Virus (PRRSV) by in Situ Hybridization in Pigs Infected with Isolates of PRRSV That Differ in Virulence. Vet. Pathol. 34, 39-43.
Hill, H., 1990. Overview And History Of Mystery Swine Disease (Swine Infertility And Respiratory Syndrome). In Mystery Swine Disease Communication Meeting. Denver, CO, pp. 29-31.
Kapur, V., Elam, M.R., Pawlovich,, T.M. Murtaugh, M.P. 1996. Genetic Variation In PRRS Virus Isolates In The Midwestern United States. J. Gen. Virol. 77, 1271-1276.
Key, K. F., Haqshenas, G., Guenette, D. K., Swenson, S. L., Toth, T. E., Meng, X. J., 2001. Genetic Variation And Phylogenetic Analyses Of The Orf5 Gene Of Acute Porcine Reproductive And Respiratory Syndrome Virus Isolates. Vet. Micro. 83, 249-263.
Labarque, G., Van Gucht, S., Van Reeth, K., Nauwynck, H., Pensaert, M., 2003. Respiratory Tract Protection Upon Challenge Of Pigs Vaccinated With Attenuated Porcine Reproductive And Respiratory Syndrome Virus Vaccines. Vet. Micro. 95, 187-197.
Meng, X. J., 2000. Heterogeneity of Porcine Reproductive and Respiratory Syndrome Virus: Implications for Current Vaccine Efficacy and Future Vaccine Development. Vet. Micro. 74, 309-329.
Mengeling, W. L., Lager, K. M., Vorwald, A. C., Clouser, D. F., 2003a. Comparative Safety and Efficacy of Attenuated Single-strain and Multi-strain Vaccines for Porcine Reproductive and Respiratory Syndrome. Vet. Micro. 93, 25-38.
Mengeling, W. L., Lager, K. M., Vorwald, A. C., Koehler, K. J., 2003b. Strain Specificity of the Immune Response of Pigs Following Vaccination with Various Strains of Porcine Reproductive and Respiratory Syndrome Virus. Vet. Micro. 93, 13-24
Mengeling, W. L., Lager, K. M., Vorwald, A. C., 1998. Clinical Effects of Porcine Reproductive and Respiratory Syndrome Virus on Pigs During the Early Postnatal Interval. Amer. J. Vet Res. 59, 52-55.
Murtaugh, M.P., K.S. Faaberg, J. Laber, M. Elam, and V. Kapur. 1998. Genetic Variation In The PRRS Virus. Adv. Exp. Med. Biol. 440, 787-794.
Murtaugh, M. P., Xiao, Z., Zuckermann, F., 2002. Immunological Responses Of Swine To Porcine Reproductive And Respiratory Syndrome Virus Infection. Viral Immunology. 15, 533-547.
Nodelijk, G., de Jong, M. C. M., van Leengoed, L. A. M. G., Wensvoort, G., Pol, J. M. A., Steverink, P. J. G. M., Verheijden, J. H. M., 2001. A Quantitative Assessment Of The Effectiveness Of PRRSV Vaccination In Pigs Under Experimental Conditions. Vaccine 19, 3636-3644.
Ostrowski, M,, Galeota, J.A., Jar, A.M., Platt, K.B., Osorio, F.A., Lopez, O.J. 2002. Identification Of Neutralizing And Nonneutralizing Epitopes In The Porcine Reproductive And Respiratory Syndrome Virus Gp5 Ectodomain. J. Virol. 76, 4241-4250.
Opriessnig, T., Halbur, P. G., Yoon, K. J., Pogranichniy, R. M., Harmon, K. M., Evans, R., Key, K. F., Pallares, F. J., Thomas, P., Meng, X. J., 2002. Comparison Of Molecular And Biological Characteristics Of A Modified Live Porcine Reproductive And Respiratory Syndrome Virus (PRRSV) Vaccine (Ingelvac PRRS Mlv), The Parent Strain Of The Vaccine (Atcc Vr2332), Atcc Vr2385, And Two Recent Field Isolates Of PRRSV. J. Virol. 76, 11837-11844.
Pejsak, Z., Stadejek, T., Markowska-Daniel, I., 1997. Clinical Signs And Economic Losses Caused By Porcine Reproductive And Respiratory Syndrome Virus In A Large Breeding Farm. Vet. Micro. 55, 317-322.
Reed, L., Muench, H., 1938. A Simple Method For Estimating Fifty Percent Endpoints. Am J Hyg. 27, 493-497.
Roof, M.B., Vaughn, E.M., Burkhart, K.M., Faaberg, K.S. 2003. Efficacy Of Modified Live Virus Porcine Reproductive And Respiratory Syndrome Virus Vaccines Against Heterologous Respiratory Challenge. Proc. 4th Inter. Symp. Emerging Re-emerging Pig Diseases. pp. 117-118.
Thacker, B., 2003. Clinical Manifestations Of PRRS Virus. In: Zimmerman, J., Yoon, K. J. (Eds.), 2003 PRRS Compendium Second Edition. National Pork Board, Des Moines IA USA, pp. 7-12.
Thanawongnuwech, R., Halbur, P. G., Ackermann, M. R., Thacker, E. L., Royer, R. L.,1998. Effects Of Low (Modified-live Virus Vaccine) And High (Vr-2385)-virulence Strains Of Porcine Reproductive And Respiratory Syndrome Virus On Pulmonary Clearance Of Copper Particles In Pigs. Vet. Pathol. 35, 398-406.
Yuan, S., Mickelson, D., Murtaugh, M. P., Faaberg, K. S., 2001. Erratum To "Complete Genome Comparison Of Porcine Reproductive And Respiratory Syndrome Virus Parental And Attenuated Strains". Virus Research. 79, 189-200.

## Claims

1. Porcine reproductive and respiratory syndrome (PRRS) virus isolate MN 184 (ATCC No. PTA 6319), and attenuated forms thereof.

2. An immunogenic composition comprising an attenuated PRRS virus isolate and a pharmacologically compatible carrier, said attenuated isolate being an attenuated form of MN 184 (ATCC No. PTA 6319).

3. The immunogenic composition of claim 2 which is a modified live vaccine.
